# FASCICULE DE BREVET EUROPEEN

(11) **EP 1 841 459 B1**
(45) Date de publication et mention de la délivrance du brevet: **16.03.2011**
(21) Numéro de dépôt: 05849273.7
(22) Date de dépôt: 16.12.2005
(51) Int. Cl.: A61K 45/06, A61K 31/4985, A61P 29/00

(54) **UTILISATION D'UN DERIVE DE DIHYDROIMIDAZOPYRAZINE POUR TRAITER OU PREVENIR LA DOULEUR**
VERWENDUNG EINES DIHYDROIMIDAZOPYRAZIN-DERIVATS ZUR BEHANDLUNG ODER PRÄVENTION VON SCHMERZEN
USE OF A DIHYDROIM1DAZOPYRAZINE DERIVATIVE FOR TREATING OR PREVENTING PAIN

(30) Priorité: 17.12.2004 FR 0413453
(43) Date de publication de la demande: 10.10.2007
(73) Titulaire: IPSEN PHARMA, 92100 Boulogne-Billancourt (FR)
(72) Inventeur: AUGUET, Michel, F-91120 Palaiseau (FR); FAVRE, Christine, F-91530 Saint Maurice Montcouronne (FR); PREVOST, Grégoire, F-92160 Antony (FR); CHABRIER de LASSAUNIERE, Pierre-Etienne, F-75016 Paris (FR)
(74) Mandataire: Audonnet, Nathalie
(86) Numéro de dépôt international: PCT/FR2005/003160
(87) Numéro de publication internationale: WO 2006/067310

(56) Documents cités:
- EP-A1- 2 123 626
- WO-A-00/02881
- WO-A-01/49322
- WO-A-97/30053
- WO-A-2004/110415
- WO-A-2005/000852

## Description

La présente invention a pour objet l'utilisation d'un dérivé de dihydroimidazopyrazine, à savoir le (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine ou un de ses sels pharmaceutiquement acceptables pour préparer un médicament destiner à prévénir ou traiter la douleur provoquée par une neuropathie et/ou par une inflammation.

Il existe de nombreux composés présentant un effet analgésique, dont le plus connu est la morphine.

Or certains patients traités avec ces composés peuvent présenter une « tolérance », voire une résistance au produit administré, qui se manifeste par une baisse d'éfficacité du composé. Dans ce cas il est nécessaire d'augmenter les doses administrées ce qui peut provoquer des effets secondaires.

Afin de répondre aux exigences des industriels il est devenu nécessaire de trouver de nouveaux composés pour préparer un médicament destiner à prévénir ou traiter la douleur. WO 2005 000 852 décrit l'utilisation du composé défini ci-dessus en tant qu'agent anti-cancer et anti-douleur dans des traitements du cancer.

Aussi le problème que se propose de résoudre l'invention est de fournir un nouveau moyen adapté pour préparer un médicament destiner à prévénir ou traiter la douleur provoquée par une neuropathie et/ou par une inflammation.

De manière inattendue, les inventeurs ont mis en évidence qu'il est possible d'utiliser le (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine ou un de ses sels pharmaceutiquement acceptables pour préparer un médicament destiné à prévénir ou à traiter la douleur provoquée par une neuropathie et/ou par une inflammation.

Dans ce but la présente invention propose l'utilisation du (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine ou un de ses sels pharmaceutiquement acceptables pour préparer un médicament destiné à prévénir ou à traiter la douleur provoquée par une neuropathie et/ou par une inflammation.

L'invention offre des avantages déterminants, en particulier l'utilisation du composé selon l'invention ne provoque pas ou peu d'effets sédatifs.

De plus, avantageusement, le composé selon l'invention étant un inhibiteur des protéines G hétérodimériques, son utilisation selon l'invention permet de s'opposer aux effets des multiples médiateurs impliqués dans la douleur provoquée par une neuropathie et/ou par une inflammation.

D'autres avantages et caractéristiques de l'invention apparaîtront clairement à la lecture de la description et des exemples donnés à titre illustratifs qui vont suivre.

L'invention concerne l'utilisation du (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine ou un de ses sels pharmaceutiquement acceptables pour préparer un médicament destiné à prévénir ou à traiter la douleur provoquée par une neuropathie et / ou par une inflammation.

Pour la suite de l'exposé, et pour faciliter la lecture de ce texte le (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine est dénommé composé (I).

Par le terme « douleur » au sens de la présente invention, il faut comprendre « l'expérience désagréable émotionnelle et sensorielle associée à un dommage tissulaire présent ou potentiel ou décrite par le patient en de tels termes » (définition selon l'International Association for the Study of Pain (IASP)). Par la suite, dans la présente demande, le terme douleur est utilisé indépendamment pour désigner la douleur ou la nociception.

Par neuropathie, on entend au sens de l'invention une altération des fonctions ou modifications pathologiques touchant le système nerveux périphérique.

Plus particulièrement la douleur traitée selon l'invention est provoquée par un traitement anti-cancéreux, comme par exemple un traitement par chimiothérapie ou radiothérapie.

Enfin, l'utilisation selon l'invention permet entre autres également de prévénir ou de traiter la douleur liée à une inflammation chronique ou non, à des maladies chroniques autres qu'un cancer, à des radiculopathies, à l'adiposis dolorosa, à la migraine, à des maladies chroniques, à la fibromyalgie, à l'algoneurodystrophie ou syndrome douloureux régional complexe, à des accidents vasculaires cérébraux, à l'arthrose, à l'arthrite, aux neuropathies diabétiques ou consécutive au HIV, à la sciatique, aux contractions musculaires douloureuses, à une intoxication, aux brûlures, aux neuralgies post herpétiques, aux lésions thalamiques ou à la sclérose en plaques.

Enfin, l'utilisation selon l'invention permet de prévénir ou de traiter la ou les douleurs provoquées par une lésion du derme ou par les douleurs pré- et/ou postopératoires par exemple les douleurs physiques : traumatismes ou amputations.

Le composé **(I)** ou son sel utilisé selon l'invention peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Le composé **(I)** ou son sel utilisé selon l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration du composé **(I)** ou son sel utilisé selon l'invention pourra se faire par voie topique, orale (p.o.), parentérale, par injections intraveineuses (i.v.), intramusculaires (i.m.), sous-cutanées (s.c.) etc.

La dose d'un produit selon la présente invention, à prévoir pour le traitement des douleurs mentionnées ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".
La figure 1 présente l'effet du composé **(I)** suite à l'injection par voie intra-veineuse dans le modèle d'hyperalgésie inflammatoire induite par la carragénine.
La figure 2 présente l'effet suite à l'injection par voie intra-veineuse du composé **(I)** dans le modèle de neuropathie induite par lésion du nerf sciatique.
Les figures 3 et 4 présentent l'effet suite à l'injection par voie intra-veineuse ou intra-péritonéale du composé **(I)** dans le modèle de neuropathie induite par administration d'un agent anti-cancéreux.

Les exemples suivants illustrent l'invention.

### Etude pharmacologique des produits de l'invention

Pour rappel dans tout le texte y compris les exemples ci-après, on désigne le (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexy)méthyl)-2-phényl-5,6-dihydroimidazo[1,2-*a*]pyrazin-7(8*H*)-yl]-2-oxoéthylamine par « Composé **(I)** ».

Le composé **(I)** répond à la formule suivante :

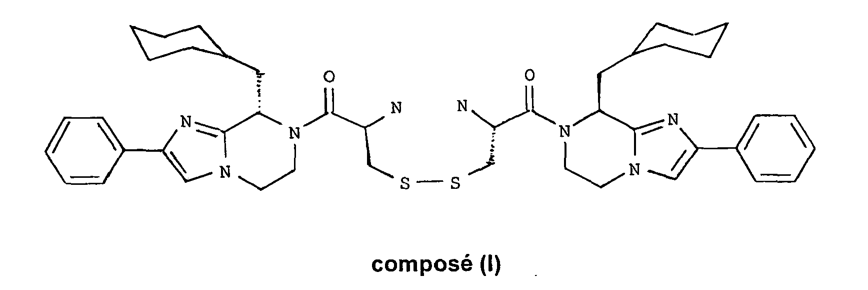

### EXEMPLE 1: modèle d'hyperalgésie inflammatoire induite par la carragénine

L'activité du composé **(I)** ou son sel selon l'invention a été évaluée in vivo sur un modèle d'hyperalgésie inflammatoire induite par injection de carragénine dans la patte de rat.

Des rats mâles Sprague Dawley (Charles River) de 200 g environ sont laissés en stabulation 5 à 8 jours dans les conditions d'animalerie et sont mis à jeun sur grilles 18 h avant et pendant l'expérience. 4 groupes sont constitués d'au moins 6 animaux. Les produits sont administrés par voie intraveineuse (i.v., 1 ml/kg) 2 h 30 après l'injection de carragénine. La carragénine à la concentration de 2 mg/0,1ml a été injectée par voie sous-plantaire dans la patte arrière droite des rats (0,1 ml ont été injecté par animal). Le seuil de la douleur (ou seuil nociceptif) a été évalué en mesurant le retrait de la patte du rat suscité par un stimulus mécanique dont la pression est croissante (pression initiale de 210 g/mm²) appliqué à l'aide d'un analgésie-mètre (test de Randall & Selitto). Les mesures ont été faites juste avant l'injection de carragénine (t = - 2 h 30) et ensuite aux temps 0,5h, 2h30 et 4h après l'injection des produits à tester, qui a été effectuée à t = 0.

L'efficacité des produits est évaluée par leur capacité à réduire de manière significative l'hyperalgésie induite par la carragénine. Cette efficacité sera statistiquement déterminée par un test d'analyse de variance (une voie) et/ou d'un test de Dunnett (deux voies).

### Effet du Composé (I) sur l'hyperalgésie induite par la carragénine:

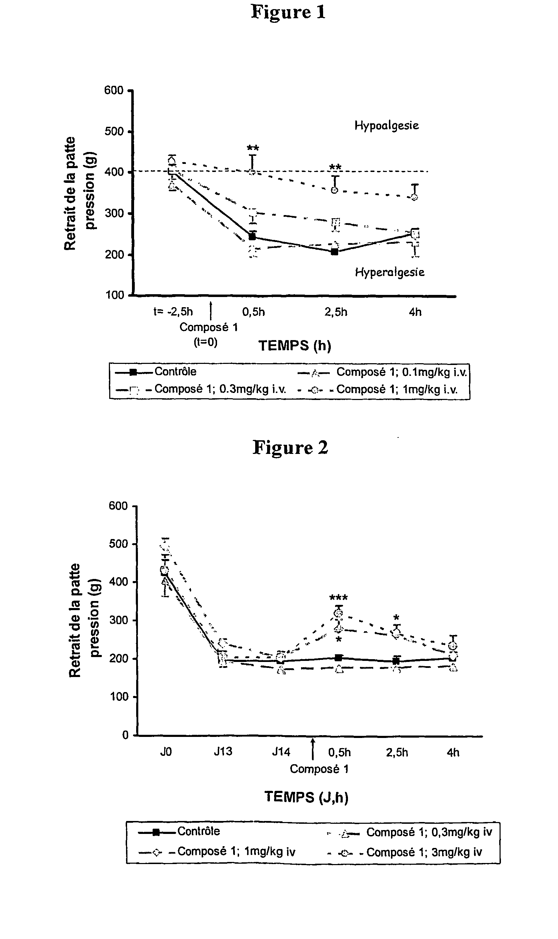

Les résultats obtenus en utilisant différentes doses du composé **(I)** dans le modèle d'hyperalgésie induite par la carragénine sur la patte de rat décrit ci-dessus sont reportés dans la figure 1.

A la figure 1, le contrôle indique le seuil de douleur toléré par le rat sur sa patte enflammée lorsque l'on applique une pression croissante. Au moment de l'injection de la carragénine ce seuil se situe entre 370 et 430 g/mm² puis diminue indiquant que plus la patte est enflammée et douloureuse moins le rat supporte l'application d'une pression sur celle-ci.

L'effet du composé **(I)** indique que le seuil de douleur toléré par le rat sur sa patte enflammée augmente plus le composé **(I)** est administré à des doses importantes. A partir de la dose de 1 mg/kg (i.v.) du composé **(I)**, le seuil de douleur suite à un stimulus mécanique appliqué sur les pattes des rats, est significativement augmenté pour atteindre environ 400 g/mm² contre 240 g/mm² pour le contrôle au temps 0,5h. Ceci permet de démontrer l'activité analgésique dose-dépendante du composé **(I)** dans ce test d'hyperalgésie induite par la carragénine.

La dose éfficace (ED₅₀) a été déterminée. Par ED₅₀, on entend la dose réduisant de moitié la douleur induite dans le modèle testé.

L'ED₅₀ est de 0,42 mg/kg au temps 0,5 h dans le modèle d'hyperalgésie induite par la carragénine.

### EXEMPLE 2 : Modèle de neuropathie induite par lésion du nerf sciatique (ou « chronic constriction injury » en anglais)

L'activité du composé **(I)** ou son sel selon l'invention a été évaluée in vivo sur un modèle de neuropathie induite par lésion du nerf sciatique sur la patte de rat.

Des rats mâles Sprague Dawley (Charles River) de 200 g environ sont laissés en stabulation pendant 6 jours dans les conditions d'animalerie. Avant le début de l'expérience (jour zéro) le seuil de la douleur (ou seuil nociceptif) a été évalué en mesurant le retrait de la patte du rat suscité par un stimulus mécanique (pression initiale de 210 g/mm²) appliqué à l'aide d'un analgésie-mètre (test de Randall & Selitto). Ce seuil est le seuil de base de la douleur. Puis, toujours au jour zéro, les rats sont rendus neuropathiques par lésion du nerf sciatique selon la méthode de Bennett and Xies (1988) : 4 ligatures espacées d'environ 2 mm (fil monocryl : 5.0) sont effectuées sur le nerf sciatique de la patte droite du rat, au-dessus de la trifurcation. Cette opération est réalisée sous anesthésie générale à l'isoflurane. Au 13^{ème} jour après l'opération de chirurgie, on sélectionne parmi les 32 rats opérés ceux qui sont les plus sensibles à la douleur en mesurant le retrait de la patte du rat suscité par un stimulus mécanique dont la pression est croissante (pression initiale de 210 g/mm²) appliqué à l'aide d'un analgésie-mètre (test de Randall & Selitto). Les résultats permettent de sélectionner 28 rats les plus sensibles, 4 groupes sont constitués de 7 animaux. Au jour 14 après l'opération, le seuil de la douleur a été ré-évalué. Puis le composé **(I)** est administré par voie intraveineuse (i.v.) à 3 concentrations différentes. Les mesures sont effectuées au temps suivant 0,5h, 2,5h et 4 h après l'injection du composé **(I).** Un groupe contrôle est réalisé par injection du véhicule (solution Nacl à 0,9% en g/100 ml) dans les mêmes conditions que le produit à tester.

L'efficacité des produits est évaluée par leur capacité à réduire de manière significative la douleur induite par lésion du nerf sciatique. Cette efficacité sera statistiquement déterminée par un test d'analyse de variance (une voie) et/ou d'un test de Dunnett (deux voies).

### Effet du Composé (I) sur la neuropathie induite par lésion du nerf sciatique:

Les résultats obtenus en utilisant différentes doses du composé **(I)** dans le modèle de neuropathie induite par lésion du nerf sciatique sur la patte de rat décrit ci-dessus sont reportés dans la Figure 2.

Sur la figure 2, le contrôle indique le seuil de douleur toléré par le rat sur sa patte lésée lorsque l'on applique une pression croissante. Au jour zéro (avant lésion) ce seuil se situe vers 420 g/mm² puis diminue pour atteindre environ 200 g/mm² au jour 13. Ces résultats indiquent que lorsque la patte est lésée celle-ci devient douloureuse et le rat supporte plus difficilement l'application d'une pression sur celle-ci.

L'administration du composé **(I)** indique que le seuil de douleur toléré par le rat sur sa patte lésée augmente proportionnellemnt aux doses du composé **(I)**. A partir de la dose de 3 mg/kg (i.v.) du composé **(I)**, le seuil de douleur suite à un stimulus mécanique appliqué sur les pattes des rats, est significativement augmenté pour atteindre environ 320 g/mm² contre 200 g/mm² pour le contrôle au temps 0,5h. Ceci permet de démontrer l'activité analgésique dose dépendante du composé **(I)** dans ce test de neuropathie induite par lésion du nerf sciatique.

La dose éfficace (ED₅₀) a été déterminée. Par ED₅₀, on entend la dose réduisant de moitié la douleur induite dans le modèle testé.

L'ED₅₀ est de 2,5 mg/kg au temps 0,5 h dans le modèle de neuropathie induite par lésion du nerf sciatique.

### EXEMPLE 3 : modèle de neuropathie induite par administration d'un agent anti-cancéreux.

L'activité du composé **(I)** ou son sel selon l'invention a été évaluée in vivo sur un modèle de neuropathie induite par administration d'un agent anti-cancéreux (paclitaxel (taxol)), sur la patte de rat.

Des rats mâles Sprague Dawley (Charles River) de 200 g environ sont laissés en stabulation pendant 6 jours dans les conditions d'animalerie. 3 groupes sont constitués d'au moins 10 animaux.

La neuropathie est induite par injections intra-péritonéales (i.p.) de 2mg/kg de paclitaxel (extrait de Taxus Yannanensis) aux jours J0, J2, J4 et J7.

Avant la première injection, les rats sont numérotés et pesés et la nociception est évaluée après un stimulus mécanique dont la pression est croissante : induction d'une pression initiale (210g/m²) sur les deux pattes arrières des rats effectuée à l'aide d'un analgésie-mètre selon la méthode de Randall et Selitto. Ces mesures permettent de définir les valeurs basales avant le développement de la neuropathie (J0).

La diminution du seuil nociceptif correspondant à l'atteinte neuropathique est maximale entre le 16^{éme} et le 24^{éme} jour après la première injection de paclitaxel. Le seuil de nociception des deux pattes arrières des rats est diminué semblablement permettant de moyenner les valeurs pour faciliter les calculs. Les études de protection de la neuropathie seront donc effectuées entre le 16^{éme} et le 24^{éme} jour.

Le jour de l'expérience, les rats sont pesés, la nociception est mesurée et les animaux n'ayant pas développés la neuropathie ce jour (diminution de la nociception par rapport aux mesures à J0) sont exclus de l'étude. Le produit testé est administré et la nociception est mesurée à différents temps après son administration.

### Effet du Composé (I) sur la neuropathie induite par le taxol:

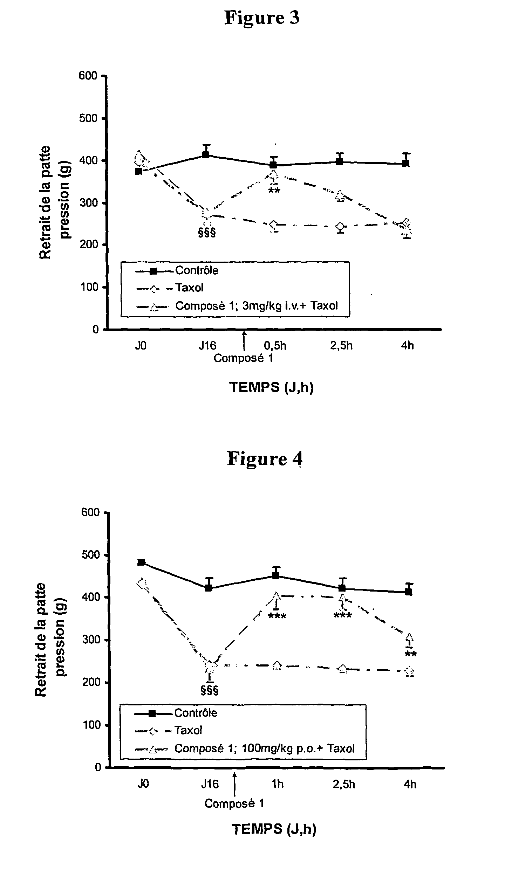

Les résultats obtenus en utilisant différentes doses du composé **(I)** dans le modèle de neuropathie induite par le taxol décrit ci-dessus sont reportés dans la Figure 3.

Sur la figure 3, le contrôle indique le seuil de douleur toléré par le rat lorsque l'on applique une pression croissante sur ses pattes. Au jour zéro ce seuil se situe vers 400 g/mm² puis diminue pour atteindre environ 270 g/mm² au jour 16. Ces résultats indiquent qu'après injection i.p. de paclitaxel la sensibilité des pattes des rats est augmentée suite à l'application d'une pression sur celles-ci.

L'administration du composé **(I)** indique que le seuil de douleur toléré par le rat sur ses pattes après induction de la neuropathie par le paclitaxel augmente avec le composé **(I)** à la dose de 3 mg/kg (i.v.). Le seuil de douleur suite à un stimulus mécanique appliqué sur les pattes des rats, est significativement augmenté pour atteindre environ 370 g/mm² contre 250 g/mm² pour le paclitaxel seul au temps 0,5h. Ceci permet de démontrer l'activité analgésique du composé **(I)** dans ce test de neuropathie induite par injection systémique chronique de paclitaxel.

Sur la figure 4, le contrôle indique le seuil de douleur toléré par le rat lorsque l'on applique une pression croissante sur ses pattes. Au jour zéro ce seuil se situe vers 450 g/mm² puis diminue pour atteindre environ 235 g/mm² au jour 16. Ces résultats indiquent qu'après injection i.p. de paclitaxel la sensibilité des pattes des rats est augmentée suite à l'application d'une pression sur celles-ci.

L'administration du composé **(I)** indique que le seuil de douleur toléré par le rat sur ses pattes après induction de la neuropathie par le paclitaxel augmente avec le composé **(I)** à la dose de 100 mg/kg (p.o.). Le seuil de douleur suite à un stimulus mécanique appliqué sur les pattes des rats, est significativement augmenté pour atteindre environ 400 g/mm² contre 240 g/mm² pour le paclitaxel seul au temps 1 h. Ceci permet de démontrer l'activité analgésique du composé **(I)** dans ce test de neuropathie induite par injection systémique chronique de paclitaxel.

## Revendications

1. Utilisation du (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine ou un de ses sels pharmaceutiquement acceptables pour préparer un médicament destiné à prévenir ou à traiter la douleur, **caractérisée en ce que** la douleur est provoquée par une neuropathie et / ou par une inflammation.

2. Utilisation selon la revendication 1 **caractérisée en ce que** que la douleur est provoquée par un traitement anti-cancéreux.

3. Utilisation selon la revendication 2 **caractérisée en ce que** que le traitement anti-cancéreux est un traitement par chimiothérapie ou radiothérapie.

4. Utilisation selon l'une des revendications précédentes **caractérisée en ce que** la douleur est liée à une inflammation chronique ou non, à des maladies chroniques autres qu'un cancer, à des radiculopathies, à l'adiposis dolorosa, à la migraine, à des maladies chroniques, à la fibromyalgie, à l'algoneurodystrophie ou syndrome douloureux régional complexe, à des accidents vasculaires cérébraux, à l'arthrose, à l'arthrite, aux neuropathies diabétiques ou consécutive au HIV, à la sciatique, aux contractions musculaires douloureuses, à une intoxication, aux brûlures, aux neuralgies post herpétiques, aux lésions thalamiques ou à la sclérose en plaques.

5. Utilisation selon l'une des revendications précédentes **caractérisée en ce que** la douleur est provoquée par une lésion du derme ou par les douleurs pré- et/ou postopératoires.

## Claims

1. Use of (1*R*)-1-[({(2*R*)-2-amino-3-[(8*S*)-8-(cyclohexylmethyl)-2-phenyl-5,6-dihydroimidazo[1,2-*a*]pyrazin-7(8*H*)-yl]3-oxopropyl}dithio)methyl]-2-[(8*S*)-8-(cyclohexylméthyl)-2-phenyl-5,6-dihydroimidazo[1,2-*a*]pyrazin-7(8*H*)-yl]-2-oxoethylamine or one of its pharmaceutically acceptable salts for preparing a medicament intended for the prevention or treatment of pain, **characterized in that** the pain is caused by a neuropathy and/or an inflammation.

2. Use according to claim 1, **characterized in that** the pain is caused by an anti-cancer treatment.

3. Use according to claim 2, **characterized in that** the anti-cancer treatment is a treatment by chemotherapy or radiotherapy.

4. Use according to any one of the preceding claims, **characterized in that** the pain is associated with an inflammation, chronic or otherwise, chronic diseases other than cancer, radiculopathies, adiposis dolorosa, migraine, chronic diseases, fibromyalgia, algoneurodystrophy or complex regional pain syndrome, cerebrovascular accidents, arthrosis, arthritis, diabetic neuropathies or following HIV, sciatica, painful muscular contractions, intoxication, burns, post- herpetic neuralgias, thalamic lesions or multiple sclerosis.

5. Use according to any one of the preceding claims, **characterized in that** the pain is caused by a dermal lesion or by pre- and/or post-operative pain.

## Patentansprüche

1. Verwendung von (1R)-[({(2R)-2-Amino-3-[(8S)-8-(cyclohexylmethyl)-2-phenyl-5,6-dihydroimidazol[1,2-a]pyrazin-7(8H)-yl]-3-oxopropyl}dithio)methyl]-2-[(8S)-(cyclohexylmethyl)-2-phenyl-5,6-dihydroimidazo[1,2-a}pyrazin-7(8H)-yl]-2-oxoethylamin oder eines seiner pharmazeutisch annehmbaren Salze zur Herstellung eines Medikaments, das dazu bestimmt ist, Schmerz zu verhindern oder zu behandeln, **dadurch gekennzeichnet, dass** der Schmerz durch eine Neuropathie und/oder eine Entzündung hervorgerufen wird.

2. Verwendung nach Anspruch 1, **dadurch gekennzeichnet, dass** der Schmerz durch eine Antikrebsbehandlung hervorgerufen wird.

3. Verwendung nach Anspruch 2, **dadurch gekennzeichnet, dass** die Antikrebsbehandlung eine Behandlung durch Chemotherapie oder Strahlentherapie ist.

4. Verwendung nach einem der vorangehenden Ansprüche, **dadurch gekennzeichnet, dass** der Schmerz mit einer chronischen oder nicht-chronischen Entzündung, anderen chronischen Erkrankungen als Krebs, Radiculopathien, Adipositas dolorosa, Migräne, chronischen Erkrankungen, Fibromyalgie, Algoneurodystrophie oder komplexem regionalen Schmerzsyndrom, cerebrovaskulären Ereignissen, Arthrose, Arthritis, diabetischen Neuropathien oder Neuropathien als Folge von AIDS, Ischiassyndrom, schmerzhaften Muskelkontraktionen, einer Intoxikation, Verbrennungen, Post-Herpes-Neuralgien, thalamischen Läsionen oder multipler Sklerose verbunden ist.

5. Verwendung nach einem der vorangehenden Ansprüche, **dadurch gekennzeichnet, dass** der Schmerz durch eine Verletzung der Haut oder durch pre- und/oder postoperative Schmerzen hervorgerufen wird.
